# DEMANDE DE BREVET EUROPEEN

(11) **EP 1 277 437 A1**
(43) Date de publication de la demande: **22.01.2003**
(21) Numéro de dépôt: 02291575.5
(22) Date de dépôt: 25.06.2002
(51) Int. Cl.: A61B 5/103

(54) **Dispositif permettant de déterminer le degré d'une caractéristique de la typologie corporelle**

(30) Priorité: 09.07.2001 FR 0109090
(71) Demandeur: L'OREAL, 75008 Paris (FR)
(72) Inventeur: Bazin, Roland, 91570 Bievres (FR); Giron, Franck, 77164 Ferrieres-en-Brie (FR)
(74) Mandataire: Tanty, François

(57) **Abrégé**

L'invention concerne un dispositif permettant de déterminer le degré d'une caractéristique de la typologie corporelle, notamment une caractéristique d'apparence de la peau ou des cheveux, comportant :
a) (i) un atlas comprenant une pluralité d'images de référence ,
   ou (ii) des moyens permettant d'afficher des images de référence,
   et
b) un instrument optique portatif (10') permettant d'observer directement une zone de la peau ou des cheveux, comportant des moyens agencés pour produire, dans des conditions d'illumination prédéfinies, une image de la zone examinée pouvant être comparée à l'une desdites images de référence.

## Description

La présente invention est relative à l'évaluation de caractéristiques de la typologique corporelle, notamment des caractéristiques d'apparence telles que la brillance, la couleur et le relief cutané, par exemple.

Il existe des dermatoscopes qui contiennent un système de grossissement et des moyens d'éclairage intégrés permettant de faire des observations précises. Toutefois, ces dermatoscopes ne sont pas prévus pour délivrer une image autre qu'un simple agrandissement de la zone observée. De plus, les caractéristiques de l'éclairage sont susceptibles de varier d'un dermatoscope à l'autre, ce qui n'est pas trop gênant lorsqu'il s'agit d'observer un défaut de la peau mais n'est pas satisfaisant lorsqu'il s'agit d'évaluer des caractéristiques d'apparence telles que la brillance ou la couleur, par exemple.

Des systèmes complexes utilisant des caméras vidéo ou d'autres capteurs électroniques existent par ailleurs, étant décrits par exemple dans la demande de brevet EP-A-0 655 221 ou le brevet US 5 377 000. Ces systèmes sont relativement coûteux et se prêtent mal à une large diffusion afin d'équiper par exemple tous les points de vente d'un produit ou de permettre au public de s'évaluer lui-même.

Il existe par conséquent un besoin pour bénéficier d'un dispositif simple à utiliser, relativement peu coûteux, permettant une évaluation des caractéristiques de la typologie corporelle, notamment des caractéristiques d'apparence de la peau ou des cheveux, par exemple des caractéristiques liées à l'éclat du teint ou à l'éclat des cheveux, telles que la brillance et la couleur.

L'invention vise notamment à répondre à ce besoin.

Elle y parvient grâce à un nouveau dispositif permettant de déterminer le degré d'une caractéristique de la typologie corporelle, notamment une caractéristique d'apparence de la peau et/ou des cheveux, ce dispositif comportant :
- un atlas comprenant une pluralité d'images de référence ou des moyens permettant d'afficher des images de référence, et
- un instrument optique portatif permettant d'observer directement une zone de la peau ou des cheveux, comportant des moyens agencés pour produire, dans des conditions d'illumination prédéfinies, une image de la zone examinée pouvant être comparée à l'une desdites images de référence.

L'instrument optique du dispositif selon l'invention permettant une observation directe, c'est-à-dire en regardant au travers de l'instrument optique la zone examinée, sans acquisition par un appareil photographique, une caméra vidéo ou d'autres types de capteurs électroniques, son coût de production peut rester compatible avec une large diffusion.

Selon un aspect de l'invention, l'instrument optique est dépourvu de moyens vidéo, et l'image n'est pas produite sur un écran vidéo.

Par ailleurs, l'observation pouvant s'effectuer dans des conditions d'illumination prédéfinies, il est possible de quantifier la caractéristique observée d'une manière relativement précise et reproductible.

Dans une réalisation particulière, l'instrument optique comporte un système optique agencé pour produire une image agrandie de la zone examinée. Il est ainsi possible d'évaluer des caractéristiques de la peau difficiles à observer à l'oeil nu, telles que par exemple sa desquamation.

Toujours dans une réalisation particulière, l'instrument optique est agencé pour permettre de réduire ou d'éliminer la brillance d'une partie au moins de la zone examinée, ce qui peut permettre par exemple d'observer plus facilement la couleur de la peau ou des cheveux, sans être gêné par des reflets. La suppression ou la diminution de la brillance peut également être utile pour observer la couleur de la lumière qui est rétrodiffusée, provenant des couches profondes de l'épiderme ou des cheveux, et qui dépend de l'état de ces couches. Il peut notamment être intéressant d'observer le contraste entre la lumière qui comporte une composante de réflexion et une composante rétrodiffusée et la lumière qui comporte essentiellement une composante rétrodiffusée, en éclairant par exemple la zone examinée avec une lumière polarisée et en observant avec un analyseur. Ce dernier peut comporter un polariseur rotatif ou deux polariseurs ayant des directions de polarisation différentes, dont l'une peut être perpendiculaire à la direction de polarisation de la lumière incidente et l'autre parallèle.

Dans une réalisation particulière, la zone examinée peut être éclairée à l'aide d'un moyen d'éclairage permettant de l'illuminer avec des inclinaisons différentes de la lumière incidente, afin par exemple de l'observer sous éclairage diffus ou sous éclairage rasant.

On peut disposer ainsi de plusieurs conditions d'observation, ce qui peut permettre d'évaluer plus facilement une caractéristique d'apparence, en faisant ressortir les différences entre deux images, par exemple.

L'éclairage rasant peut donner des informations de relief et l'éclairage diffus des informations sur l'homogénéité de la couleur.

Dans une réalisation particulière, l'instrument optique comporte au moins un écran apte à être interposé entre une source de lumière et la zone examinée, de manière à ne permettre l'éclairage de cette dernière que par diffusion de la lumière sous l'écran, dans le tissu examiné. Cet écran peut être mobile, pouvant être déplacé d'une première position dans laquelle il est éloigné d'une surface bordant la zone examinée à une deuxième position dans laquelle il vient au contact de ladite surface. On peut ainsi observer la peau ou les cheveux par transillumination, c'est-à-dire que l'éclairage d'une zone de peau ou de cheveux s'effectue par la lumière provenant de zones adjacentes, la peau ou les cheveux servant de guide de lumière, et obtenir notamment une information de transparence, cette information pouvant se combiner le cas échéant à d'autres informations tirées d'observations précédentes ayant eu lieu dans des conditions d'illumination différentes. L'écran peut encore être amené dans une position dans laquelle il permet un éclairage rasant de la zone examinée. L'écran peut comporter une paroi tubulaire s'étendant, au cours de l'utilisation, autour de la zone examinée. L'écran qui peut être de section non circulaire peut comporter une portion conique ou pyramidale convergeant vers la zone examinée. L'instrument optique peut comporter au moins un ressort pour rappeler l'écran dans une position de repos, en l'absence d'utilisation.

L'instrument optique peut comporter un réticule, permettant notamment de mesurer la distance à partir de laquelle la diffusion de la lumière n'est plus visible.

Dans une réalisation particulière, l'instrument optique comporte au moins un filtre coloré. Ce filtre peut être de couleur bleue, afin de faire ressortir la pigmentation de la peau, par exemple.

Dans une réalisation particulière, l'instrument optique comporte au moins un polariseur. Ce polariseur peut être placé sur le trajet de la lumière, entre une source de lumière et la zone examinée. L'instrument optique peut également comporter au moins un polariseur placé sur le trajet de la lumière entre la zone examinée et un oeil de l'observateur. L'instrument optique peut comporter au moins deux polariseurs d'orientations différentes, juxtaposés, placés sur le trajet de la lumière entre la zone examinée et un oeil de l'observateur. On peut ainsi observer un contraste entre deux zones de l'image, et profiter de la sensibilité plutôt élevée de l'oeil humain à un contraste. L'instrument optique peut comporter au moins un polariseur monté rotatif de manière à permettre à l'utilisateur de modifier l'orientation de sa direction de polarisation par rapport à une direction de référence.

Dans ce dernier cas, le dispositif peut comporter par exemple une poignée comportant un organe d'actionnement tel qu'une molette par exemple, permettant de modifier l'orientation du polariseur avec la même main que celle qui tient la poignée.

L'instrument optique peut être agencé pour permettre l'éclairage de la zone examinée par la lumière naturelle. L'instrument optique peut notamment comporter une jupe en matière plastique transparente, dont un bord peut s'étendre autour de la zone examinée.

L'instrument optique peut aussi comporter au moins une source lumineuse intégrée. Cette source lumineuse intégrée peut comporter au moins un élément lumineux choisi parmi les suivants : lampe à incandescence, diode électroluminescente, lampe à fluorescence. L'instrument optique peut comporter des éléments lumineux éclairant dans des gammes respectives de longueur d'onde différentes. Le dispositif peut par exemple comporter une source reproduisant les caractéristiques spectrales de la lumière naturelle, pouvant comporter des diodes électroluminescentes légèrement colorées. L'instrument optique peut comporter plusieurs éléments lumineux et des moyens de commande permettant d'alimenter sélectivement au moins une partie de ces éléments lumineux. L'instrument optique peut comporter des éléments lumineux disposés circulairement.

L'instrument optique peut comporter un logement pour recevoir une ou plusieurs piles électriques. Un tel logement peut présenter un axe sensiblement perpendiculaire à une direction d'observation de la zone examinée.

L'instrument optique peut comporter une vitre permettant de comprimer la peau dans la zone examinée pour en chasser le sang. Une telle vitre peut être réalisée en verre ou en matière plastique transparente, éventuellement colorée. Une telle vitre peut constituer un accessoire amovible ou un élément fixé à demeure sur l'instrument, mobile entre une position active dans laquelle elle s'interpose entre la peau et le trajet de la lumière vers l'observateur et une position inactive, dans laquelle la vitre ne se situe pas sur le trajet de la lumière vers l'observateur.

L'instrument optique peut comporter une bague permettant l'adaptation d'un appareil photographique.

Dans une réalisation particulière, le dispositif comporte un atlas comprenant une pluralité d'images de référence. Ces images peuvent être disposées sur un support unique ou être reliées ensemble. Les images peuvent être associées chacune à une indication alphanumérique.

Le dispositif peut également comporter un ordinateur pour afficher des images de référence.

L'invention a encore pour objet un procédé pour évaluer le degré d'une caractéristique de la typologie corporelle, caractérisé par le fait qu'il comporte les étapes suivantes :
a) observer la peau ou les cheveux au moyen d'un instrument optique tel que défini plus haut,
b) comparer l'image observée avec des images de référence,
c) sélectionner une image de référence.

Dans une mise en oeuvre particulière du procédé, on observe le contraste entre la lumière qui comporte une composante de réflexion et une composante rétrodiffusée et la lumière qui comporte essentiellement une composante rétrodiffusée, en éclairant la zone examinée avec une lumière polarisée et en l'observant avec un analyseur.

Les images de référence peuvent être affichées à l'écran d'un ordinateur, comme indiqué plus haut.

Les images de référence peuvent notamment être transmises à l'ordinateur depuis un serveur par un réseau informatique, avant d'être affichées sur ledit écran.

Le procédé peut comporter en outre l'étape consistant à transmettre au serveur une indication représentative de l'image de référence sélectionnée. Le serveur peut alors être programmé de manière à établir un diagnostic, par exemple, ou préconiser un produit cosmétique ou de soins.

Dans une mise en oeuvre particulière de l'invention, on procède à une évaluation d'une caractéristique de la typologie corporelle, on effectue un traitement, notamment cosmétique, susceptible d'avoir une incidence sur ladite caractéristique, puis on procède à une nouvelle évaluation pour détecter une éventuelle évolution de ladite caractéristique et déterminer l'efficacité du traitement.

Comme indiqué plus haut, on peut effectuer à l'aide du même instrument optique successivement ou simultanément au moins deux types d'observations, choisis parmi les suivants :
i) observation sous éclairage diffus,
ii) observation sous éclairage rasant,
iii) observation sous éclairage directif,
iv) observation par transillumination,
v) observation sous lumière polarisée avec un analyseur non croisé,
vi) observation sous lumière polarisée avec un analyseur croisé,
vii) observation en comprimant la peau,
viii) observation sans comprimer la peau,
ix) observation en étirant la peau,
x) observation sans étirer la peau,
xi) observation en plissant la peau,
xii) observation sans plisser la peau.

Les types d'observation choisis peuvent notamment être i) et ii), v) et vi), vii) et viii), ix) et x), xi) et xii).

Lorsque l'on observe pas transillumination, il est possible de mesurer la distance à partir de laquelle la diffusion de la lumière n'est plus visible.

D'autres caractéristiques et avantages de la présente invention ressortiront à la lecture de la description détaillée qui va suivre, d'exemples non limitatifs de mise en oeuvre, et à l'examen du dessin annexé, sur lequel :
- la figure 1 représente de manière très schématique, en coupe axiale et partielle, un instrument optique pour la mise en oeuvre de l'invention,
- la figure 2 est une vue partielle de dessus selon la flèche II de la figure 1, illustrant le positionnement des éléments lumineux,
- la figure 3 représente le dispositif de la figure 1 dans une configuration d'observation sous éclairage rasant,
- la figure 4 représente le dispositif de la figure 1 dans une configuration permettant une transillumination,
- la figure 5 est une coupe axiale, schématique et partielle, d'une variante du dispositif de la figure 1,
- la figure 6 représente le dispositif de la figure 5, dans une configuration permettant un éclairage rasant,
- la figure 7 représente le dispositif de la figure 5 dans une configuration permettant la transillumination,
- la figure 8 représente le polariseur du dispositif des figures 5 à 7, en vue de dessus,
- la figure 9 est une vue partielle de dessus, représentant le disque analyseur du dispositif des figures 5 à 7, et illustrant la disposition des émetteurs lumineux,
- la figure 10 représente une variante du dispositif des figures 5 à 7,
- la figure 11 représente, en coupe axiale schématique et partielle, un autre exemple d'instrument optique pour la mise en oeuvre de l'invention,
- la figure 12 est une vue partielle de dessus de la figure 11,
- la figure 13 représente le dispositif de la figure 11 dans une configuration d'éclairage rasant,
- la figure 14 représente le dispositif de la figure 11 dans une configuration permettant la transillumination,
- la figure 15 représente un autre exemple de dispositif, en perspective éclatée,
- la figure 16 est une vue partielle de face selon XVI de la figure 15,
- la figure 17 représente une variante d'une partie du dispositif de la figure 15,
- la figure 18 représente de manière très schématique un atlas de brillance,
- la figure 19 représente de manière très schématique un atlas de contrastes,
- la figure 20 représente de manière très schématique un atlas de sécheresse cutanée,
- la figure 21 illustre la réalisation d'un atlas sous la forme d'un ensemble d'images reliées,
- la figure 22 représente de manière schématique un ordinateur permettant d'afficher, sur un écran, des images de référence, et
- la figure 23 illustre la connexion d'un ordinateur, sur lequel peuvent être affichées des images de référence, à un serveur distant.

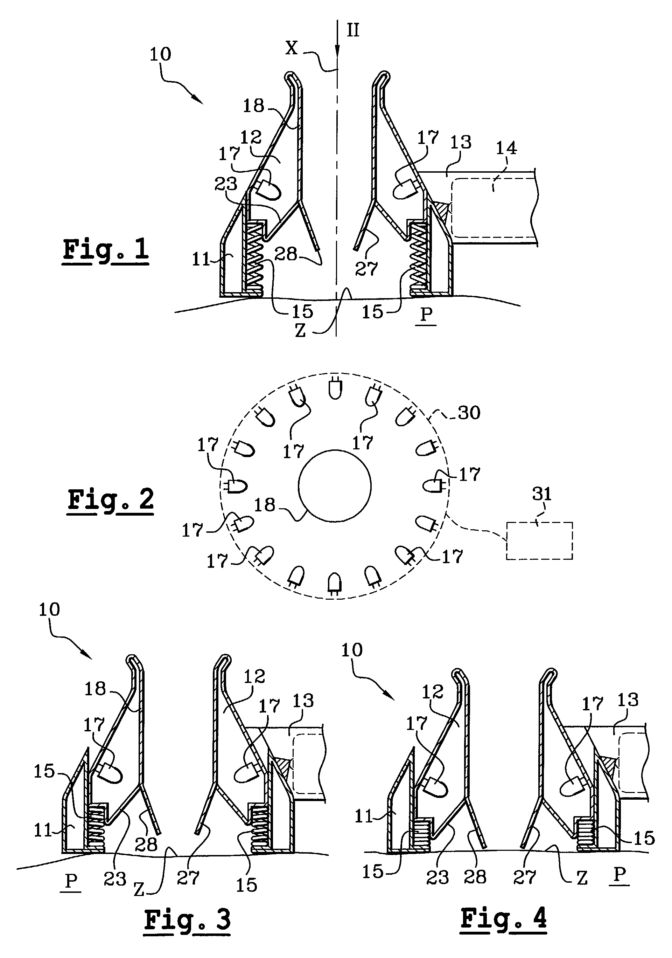

L'instrument optique 10 représenté sur les figures 1 à 4 est destiné à l'observation de la peau P ou des cheveux et comporte un support 11 à poser sur la surface à observer, un ensemble mobile 12 pouvant être déplacé par rapport au support 11 selon un axe X, et une poignée 13 permettant la préhension de l'instrument par un utilisateur, cette poignée 13 étant fixe par rapport au support 11 et logeant intérieurement une ou plusieurs piles ou accumulateurs 14.

Un système de rappel élastique interagit entre le support 11 et l'ensemble mobile 12, de manière à rappeler ce dernier dans une position de repos, correspondant à la figure 1, dans laquelle l'ensemble 12 vient en appui contre la poignée 13. Dans l'exemple illustré, ce système de rappel comporte plusieurs ressorts 15 en travaillant en compression, venant en appui à une extrémité contre un épaulement du support 11 et à l'autre extrémité contre l'ensemble 12.

Ce dernier comporte une pluralité d'émetteurs lumineux 17 constitués, dans l'exemple décrit, par des diodes électroluminescentes blanches, réparties tout autour de l'axe X, comme illustré sur la figure 2, de manière à produire soit un éclairage diffus de la zone Z examinée lorsque tous les émetteurs lumineux sont alimentés, soit un éclairage dans une ou plusieurs directions particulières, si seulement une partie des émetteurs lumineux est alimentée. Les émetteurs lumineux 17 sont alimentés en électricité grâce aux piles 14 par un circuit d'alimentation 30, représenté très schématiquement, dans un souci de clarté du dessin. Ce circuit d'alimentation 30 est relié à un circuit de commande 31.

L'observation s'effectue au travers d'un tube 18 de l'ensemble mobile 12.

Un élément optique 23 est placé sur le trajet de la lumière entre les émetteurs lumineux 17 et la zone examinée Z. Cet élément optique 23 est constitué dans l'exemple considéré par une pièce annulaire translucide, qui peut être incolore ou colorée, et qui constitue un diffuseur.

Le tube 18 est prolongé inférieurement par un écran 27, dont le bord libre 28 délimite le champ d'observation.

L'instrument optique 10 permet d'effectuer des observations successives, dans des conditions d'éclairage différentes, de la même surface.

Dans la configuration représentée à la figure 1, la zone examinée Z peut être éclairée de manière diffuse et omnidirectionnelle par la totalité des émetteurs lumineux 17.

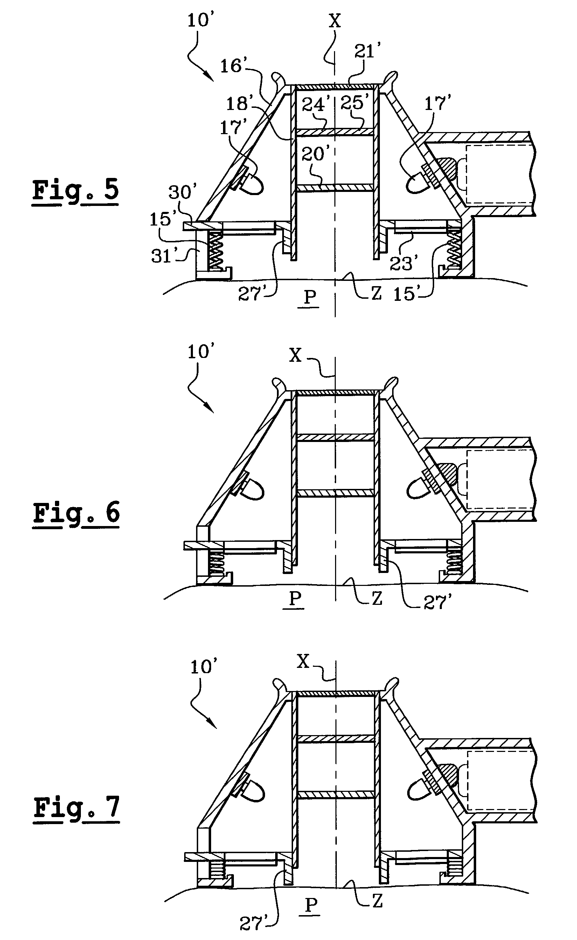

L'écran 27 peut être rapproché de la zone examinée Z grâce à la mobilité de l'ensemble 12 par rapport au support 11, comme illustré sur la figure 3. Sur cette figure, on voit que le bord inférieur 28 de l'écran 27 a pu être rapproché par l'utilisateur en exerçant une pression suffisante vers le bas sur l'ensemble 12, contre l'action de rappel des ressorts 15. On peut alors obtenir un éclairage rasant de la zone examinée Z, capable de faire ressortir davantage le relief de la peau ou des cheveux et d'augmenter la brillance.

L'utilisateur a également la possibilité d'appuyer complètement sur l'ensemble 12 de manière à amener l'écran 27 au contact de la surface de la peau ou des cheveux, comme illustré sur la figure 4. Dans ce cas, la zone examinée Z est éclairée uniquement par transillumination, c'est-à-dire par diffusion de la lumière dans le tissu observé, ce qui permet par exemple d'obtenir une information sur sa transparence, sa couleur ou son irrigation par le sang. Le courant d'alimentation des émetteurs lumineux 17 peut rester inchangé entre les différentes observations.

Le circuit de commande 31 peut être constitué par un simple interrupteur électrique commandant l'alimentation en tout ou rien de la totalité des émetteurs lumineux 17 ou par un système plus complexe, permettant par exemple d'alimenter sélectivement certains émetteurs lumineux 17, afin d'obtenir par exemple un éclairage directionnel ou pluridirectionnel, fixe ou tournant, voire dans le cas où les émetteurs lumineux comporteraient des diodes de différentes couleurs ou capables d'émettre à des longueurs d'ondes variables, pour modifier les caractéristiques spectrales de la lumière venant éclairer la zone examinée.

On a représenté sur les figures 5 à 7 un instrument optique 10' qui correspond à une variante de l'instrument 10 décrit précédemment.

Dans cette variante, l'instrument 10' comporte un corps 16' qui est fixe et un écran 27' qui est mobile par rapport au corps 16' selon l'axe X, pour modifier les conditions d'illumination de la zone observée Z, afin de permettre par exemple de passer d'un éclairage diffus multidirectionnel à un éclairage rasant, comme illustré à la figure 6, voire à un éclairage par transillumination, comme illustré sur la figure 7.

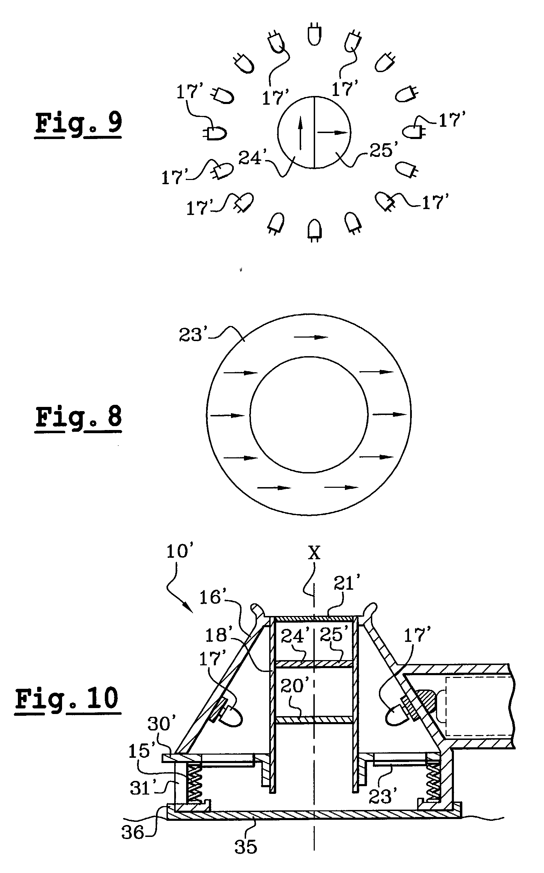

Un polariseur 23' représenté isolément sur la figure 8 est placé sur le trajet de la lumière entre les émetteurs lumineux 17' et la zone observée Z. Ce polariseur 23' présente une forme annulaire et une direction de polarisation unique, indiquée par des flèches sur la figure 8.

L'écran 27' est monté coulissant sur un tube 18' qui supporte des lentilles 20', 21' et des polariseurs 24', 25' et peut être déplacé grâce à une patte 30' faisant saillie à l'extérieur de l'instrument, traversant une fente 31' du corps 16', sur lequel sont montés les émetteurs lumineux 17'. Ces derniers sont agencés de la même manière que les émetteurs lumineux 17 de l'ensemble précédent. Un avantage de l'instrument 10' est de ne pas modifier la distance entre la zone examinée et les lentilles 20', 21' lors du changement de configuration de l'éclairage par déplacement de l'écran 27'.

Dans l'exemple considéré, deux polariseurs 24' et 25', en forme chacun de demi-disque et réunis par leur base de manière à former un disque analyseur, sont placés sur le trajet de la lumière dans le tube 18', dans le plan focal image de la lentille 20'. Les directions de polarisation des polariseurs 24' et 25' sont perpendiculaires entre elles et indiquées par des flèches sur la figure 9.

L'analyseur constitué par les polariseurs 24' et 25' permet d'obtenir une demi-image avec brillance et une demi-image sans brillance, en choisissant la direction de polarisation de l'un des demi-disques 24' et 25' parallèle à la direction de polarisation du polariseur 23'.

Le cas échéant, les polariseurs 24' et 25' sont remplacés par un polariseur monté rotatif autour de l'axe X afin de pouvoir faire d'une part coïncider sa direction de polarisation avec la direction de polarisation de la lumière incidente et d'autre part orienter sa direction de polarisation perpendiculairement à la direction de polarisation du polariseur 23'.

L'analyseur en deux parties 24', 25' permet d'obtenir une image contrastée, et donc de profiter de la sensibilité accrue de l'oeil humain à un contraste.

Chaque instrument 10 ou 10' peut recevoir, comme illustré pour l'instrument 20'sur la figure 10 une vitre 35 à travers laquelle s'effectue l'observation. Cette vitre 35 peut être réalisée en matière plastique par exemple, et comporter un rebord 36 permettant son positionnement sur l'instrument. Dans l'exemple considéré, la vitre 35 constitue un accessoire amovible mais on ne sort pas du cadre de la présente invention lorsque la vitre est escamotable et reste solidaire de l'instrument dans sa position escamotée. Lorsqu'elle est en place sur l'instrument, la vitre 35 permet de comprimer la peau et d'en chasser le sang ; on peut ainsi effectuer des observations de la peau en diminuant l'incidence de la couleur du sang sur la couleur de la peau. On peut procéder à une série d'observations sans la vitre 35, puis à une nouvelle série d'observations avec la vitre 35, sous différents types d'éclairage ou de polarisations et comparer les résultats pour en tirer une information utile.

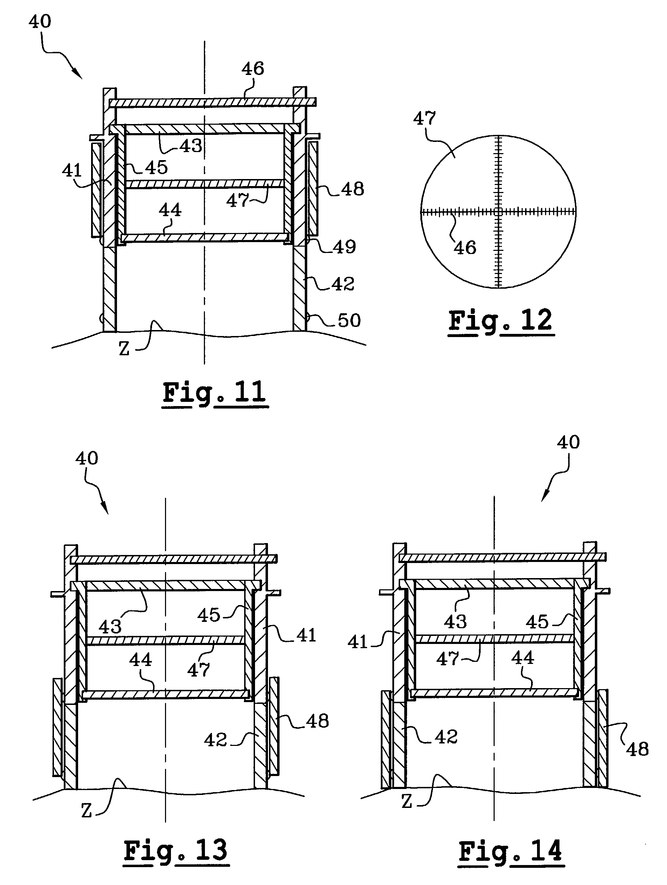

On a représenté sur les figures 11, 13 et 14 un instrument 40 dont une particularité, par rapport aux instruments 10 et 10' précédemment décrits, est de ne comporter aucun éclairage intégré.

L'instrument 40 comporte un corps 41 prolongé vers le bas par une jupe transparente 42.

Le corps 41 sert de support à un ensemble optique comportant des lentilles 43, 44 portées par un tube 45, encliqueté dans le corps 41. L'ensemble optique comporte également un réticule 46 permettant de mesurer une distance sur la zone examinée. Ce réticule est imprimé, dans l'exemple décrit et représenté isolément sur la figure 12, sur un verre 47 placé sur le trajet de la lumière provenant de la zone examinée.

Un filtre coloré 46 est monté de manière amovible sur le corps 41. Dans l'exemple représenté, ce filtre est bleu, de manière à faire ressortir, si on le souhaite, les tâches pigmentaires.

Un écran constitué par une bague opaque 48 peut coulisser sur le corps 41 et sur la jupe transparente 42, entre une position complètement escamotée, correspondant à la figure 11, dans laquelle la bague 48 ne vient pas recouvrir la jupe transparente 42, une position intermédiaire correspondant à la figure 13, dans laquelle la bague 48 vient recouvrir une majeure partie de la jupe transparente 42, afin de permettre un éclairage rasant de la zone examinée, et une position complètement descendue de la bague 48 correspondant à la figure 14, dans laquelle cette dernière recouvre en totalité la jupe transparente 42 et permet un éclairage par transillumination. Dans l'exemple représenté, des godrons 49 et 50 sont formés sur le corps 41 et la jupe transparente 42, de manière à permettre l'immobilisation de la bague 48 dans chacune des différentes configurations décrites.

Lorsque la zone examinée est observée par transillumination, l'observateur peut mesurer au moyen du réticule la distance à partir de laquelle la diffusion de la lumière n'est plus visible et en tirer une information sur la transparence de la peau.

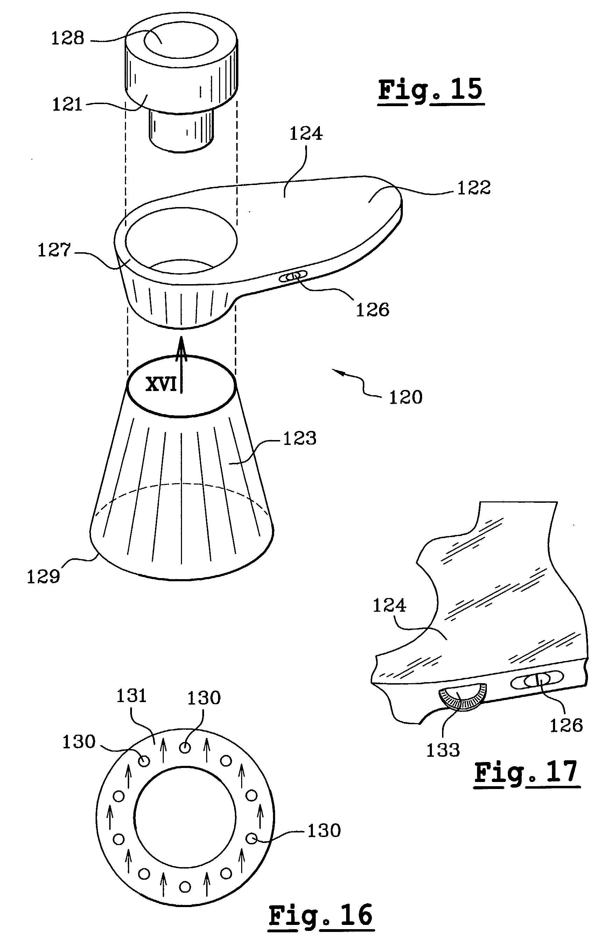

On a représenté à la figure 15 un autre exemple de dispositif d'observation 120 réalisé conformément à l'invention.

Ce dispositif 120 comporte une source lumineuse 122, un embout 123, et un ensemble optique 121.

La source lumineuse 122 comporte une poignée 124 qui forme un logement à l'intérieur duquel sont disposées une ou plusieurs piles ou accumulateurs, et une partie ajourée 127 dans laquelle peut être engagé l'ensemble optique 121.

Ce dernier comporte un oculaire 128 et une ou plusieurs lentilles non représentées, de manière à produire une image agrandie, ainsi qu'un polariseur.

La source lumineuse 122 comporte un sélecteur 126 permettant d'éclairer la zone à observer avec par exemple deux intensités lumineuses différentes ou deux types d'éclairage différents, par exemple l'un simulant la lumière du jour et l'autre un éclairage incandescent.

L'embout 123 présente une forme générale tronconique avec une base 129 ayant un diamètre supérieur au champ d'observation de l'ensemble optique 121.

Dans l'exemple illustré, le diamètre de la base 129 est de l'ordre de 40 mm et celui du champ d'observation de l'ordre de 30 mm. On évite ainsi que la pression de contact de l'embout 123 sur la peau influence l'aspect de la zone située dans le champ d'observation.

On peut prévoir une fixation magnétique de l'embout 123 sur la source lumineuse 122. L'embout 123 peut par exemple comporter un anneau aimanté métallique.

L'embout 123 peut comporter à son extrémité qui vient au contact de la peau une bague amovible.

La source lumineuse 122 comporte, comme on peut le voir sur la figure 16, une pluralité de diodes électroluminescentes 130 dont l'axe est sensiblement parallèle à celui de l'ensemble optique 121. Ces diodes électroluminescentes 130 sont recouvertes par un polariseur 131.

La hauteur de l'embout 123 est choisie de telle sorte que l'éclairage de la zone de peau située dans le champ d'observation soit sensiblement homogène.

Le dispositif d'observation 120 s'utilise de la manière suivante :
L'embout 123 et l'ensemble optique 121 étant en place sur la source lumineuse 122, l'utilisateur peut faire tourner l'ensemble optique 121 relativement à la source lumineuse de telle sorte que le polariseur contenu dans l'ensemble optique 121 ait la même direction de polarisation que le polariseur 131 ou une direction sensiblement perpendiculaire.

Il est ainsi possible d'observer la peau successivement avec brillance et sans brillance.

Dans une variante de réalisation, l'ensemble optique 121 comporte deux polariseurs juxtaposés ayant des directions de polarisation perpendiculaires, à l'instar de l'exemple de réalisation décrit précédemment, en référence à la figure 9.

Le dispositif 120 peut comporter, comme illustré à la figure 17, une molette 133 permettant à l'utilisateur d'entraîner en rotation l'ensemble optique 121 avec la main qui tient la poignée 124.

Chaque image observée au moyen d'un instrument optique, par exemple l'un de ceux qui viennent d'être décrits, cette observation ayant eu lieu dans des conditions d'illumination spécifiques, peut être comparée avec une image de référence d'un atlas comportant plusieurs images de référence, par exemple des images exprimant à des degrés divers une caractéristique de la typologie corporelle, notamment la brillance ou la couleur de la peau, ou des images correspondant à divers degrés de contraste.

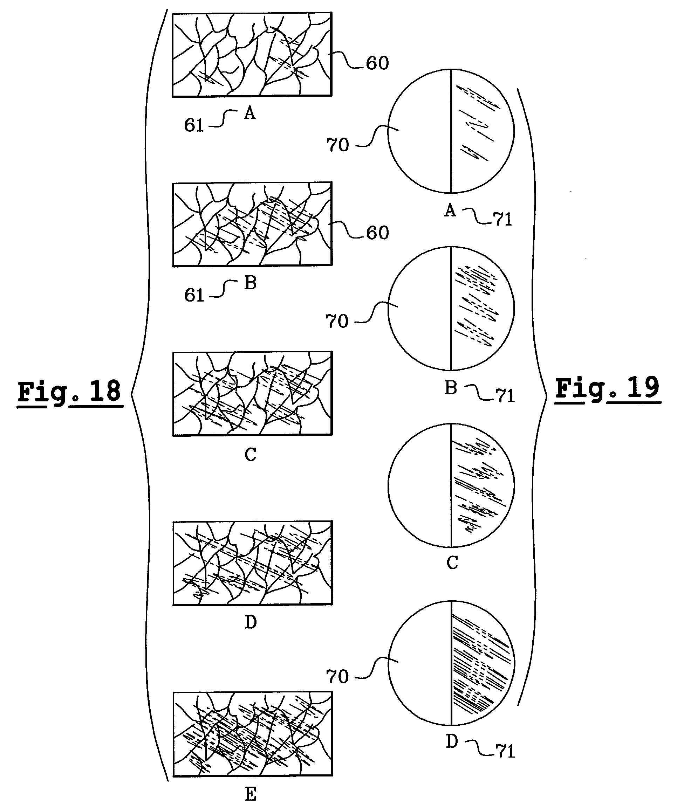

On a représenté de manière très schématique, à titre d'exemple, sur la figure 18, un atlas de brillance comportant plusieurs images de référence 60 correspondant chacune à un degré de brillance de la peau, quantifié par un identifiant alphanumérique 61.

On a représenté de manière très schématique sur la figure 19 un atlas de contraste ou d'écarts de luminosité ou de couleur, comportant plusieurs images de référence 70, correspondant à différents degrés de contraste ou d'écart pouvant être observés lors de l'utilisation d'un analyseur tel que celui formé par les demi-disques 24', 25', précédemment décrit. Ces images 70 sont associées à des identifiants alphanumériques 71, permettant de les repérer.

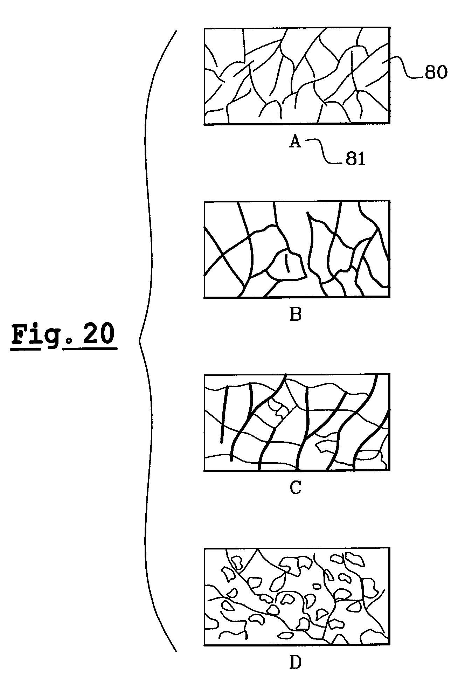

On a représenté à la figure 20 de manière très schématique un atlas de sécheresse cutanée, comportant plusieurs images 80, associées chacune à un identifiant alphanumérique 81, les images 80 exprimant divers degrés de sécheresse cutanée, allant d'une desquamation sévère, caractéristique d'une peau extrêmement sèche, à une absence de desquamation, traduisant une peau normale.

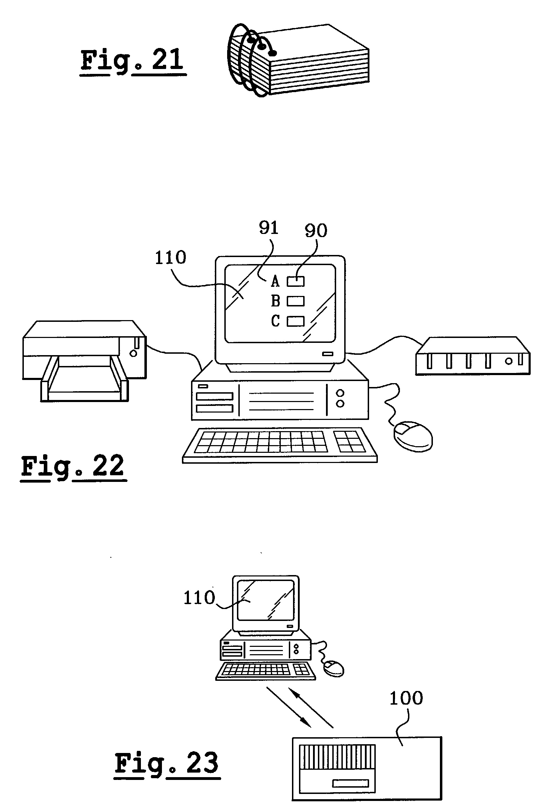

Les images de référence d'un atlas peuvent être imprimées sur un même support ou être reliées ensemble, comme illustré sur la figure 21.

Des images de référence peuvent également être affichées à l'écran d'un ordinateur 110, comme illustré à la figure 22, chaque image étant associée à un identifiant alphanumérique.

Les résultats d'une évaluation peuvent être transmis à distance par un réseau informatique, notamment le réseau Internet, au moyen de l'ordinateur 110 à un serveur 100, comme illustré à la figure 23. Le serveur 100 peut être agencé pour rendre un diagnostic en fonction des résultats transmis et, le cas échéant, préconiser un produit cosmétique ou de soins.

Dans le cas où des images de référence seraient affichées sur l'écran d'un ordinateur, ces images peuvent être transmises par le serveur 100, après connexion de l'utilisateur sur le site Internet correspondant.

L'invention peut être mise en oeuvre de manière à suivre l'efficacité d'un traitement, en procédant à une évaluation après chaque phase du traitement et en comparant les résultats des évaluations successives.

Grâce à la possibilité, dans certains modes de mise en oeuvre de l'invention, d'obtenir au moyen d'un même instrument optique des informations de brillance, de couleur, de relief et de transparence, et grâce à un atlas permettant de quantifier ces informations, on peut constituer une banque de données multivectorielle regroupant un ensemble de vecteurs correspondant chacun à un individu, chaque vecteur comportant au moins deux composantes constituées chacune par le résultat d'une observation effectuée au moyen d'un même instrument optique.

Bien entendu, l'invention n'est pas limitée aux exemples qui viennent d'être décrits et l'on peut modifier la structure de l'instrument optique, notamment en remplaçant les diodes électroluminescentes par d'autres moyens d'éclairage, tels que par exemple des lampes à incandescence ou à fluorescence. Dans le cas où un écran serait utilisé pour observer par transillumination, cet écran peut être constitué par un élément rapporté, fixé de manière amovible sur l'instrument optique.

## Revendications

1. Dispositif permettant de déterminer le degré d'une caractéristique de la typologie corporelle, notamment une caractéristique d'apparence de la peau ou des cheveux, comportant :
a) (i) un atlas comprenant une pluralité d'images de référence (60 ; 70 ; 80), ou (ii) des moyens (110) permettant d'afficher des images de référence (90), et
b) un instrument optique portatif (10 ; 10' ; 40 ; 120) permettant d'observer directement une zone de la peau ou des cheveux, comportant des moyens agencés pour produire, dans des conditions d'illumination prédéfinies, une image de la zone examinée pouvant être comparée à l'une desdites images de référence.

2. Dispositif selon la revendication précédente, **caractérisé par le fait que** l'instrument optique comporte un système optique (20' ; 41' ; 44 ; 46 ; 121) agencé pour produire une image agrandie de la zone examinée.

3. Dispositif selon l'une des deux revendications précédentes, **caractérisé par le fait que** l'instrument optique est agencé pour pouvoir soumettre la zone examinée à un éclairage diffus.

4. Dispositif selon l'une quelconque des revendications précédentes, **caractérisé par le fait que** l'instrument optique est agencé pour pouvoir soumettre la zone examinée à un éclairage directif.

5. Dispositif selon l'une quelconque des revendications précédentes, **caractérisé par le fait que** l'instrument optique est agencé pour pouvoir soumettre la zone examinée à un éclairage rasant.

6. Dispositif selon l'une quelconque des revendications précédentes, **caractérisé par le fait que** l'instrument optique comporte au moins un écran (27 ; 27' ; 48) apte à être interposé entre une source de lumière et la zone examinée, de manière à pouvoir soumettre la zone examinée à un éclairage par transillumination.

7. Dispositif selon la revendication 6, **caractérisé par le fait que** l'écran est mobile, pouvant être déplacé d'une première position dans laquelle il est éloigné d'une surface bordant la zone examinée à une deuxième position dans laquelle il vient au contact de ladite surface.

8. Dispositif selon l'une des revendications 6 et 7, **caractérisé par le fait que** l'écran peut être déplacé et amené dans une position dans laquelle il permet un éclairage rasant de la zone examinée.

9. Dispositif selon l'une quelconque des revendications 6 à 8, **caractérisé par le fait que** l'écran comporte une paroi tubulaire (27 ; 27' ; 48) s'étendant, au cours de l'utilisation, autour de la zone examinée.

10. Dispositif selon la revendication 9, **caractérisé par le fait que** l'écran comporte une portion conique (27) ou pyramidale convergeant vers la zone examinée.

11. Dispositif selon l'une quelconque des revendications 6 à 10, l'écran (27 ; 27') étant mobile, **caractérisé par le fait que** l'instrument optique comporte au moins un ressort (15 ; 15') pour rappeler l'écran dans une position de repos en l'absence d'utilisation.

12. Dispositif selon l'une quelconque des revendications précédentes, **caractérisé par le fait que** l'instrument optique comporte au moins un filtre coloré (46).

13. Dispositif selon la revendication 12, **caractérisé par le fait que** le filtre (46) est de couleur bleue.

14. Dispositif selon l'une quelconque des revendications précédentes, **caractérisé par le fait que** l'instrument optique comporte au moins un polariseur (23'; 24' ; 131)

15. Dispositif selon la revendication précédente, **caractérisé par le fait que** l'instrument optique comporte au moins un polariseur (23' ; 131) placé sur le trajet de la lumière, entre une source de lumière et la zone examinée (Z).

16. Dispositif selon l'une des deux revendications immédiatement précédentes, **caractérisé par le fait que** l'instrument optique comporte au moins un polariseur (24'; 25') placé sur le trajet de la lumière entre la zone examinée et un oeil de l'observateur.

17. Dispositif selon l'une quelconque des revendications précédentes, **caractérisé par le fait que** l'instrument optique comporte au moins deux polariseurs (24', 25') d'orientations différentes, juxtaposés, placés sur le trajet de la lumière entre la zone examinée et un oeil de l'observateur.

18. Dispositif selon l'une quelconque des revendications précédentes, **caractérisé par le fait que** l'instrument optique comporte au moins un polariseur monté rotatif de manière à permettre à l'utilisateur de modifier l'orientation de sa direction de polarisation par rapport à une direction de référence.

19. Dispositif selon l'une quelconque des revendications précédentes, **caractérisé par le fait que** l'instrument optique (40) est agencé pour permettre l'éclairage de la zone examinée par la lumière naturelle.

20. Dispositif selon la revendication précédente, **caractérisé par le fait qu'**il comporte une jupe (42) en matière plastique transparente, dont un bord peut s'étendre autour de la zone examinée.

21. Dispositif selon l'une quelconque des revendications 1 à 18, **caractérisé par le fait que** l'instrument optique comporte au moins une source lumineuse intégrée (17 ; 17' ; 122).

22. Dispositif selon la revendication 21, **caractérisée par le fait que** la source lumineuse intégrée comporte au moins un élément lumineux choisi parmi les suivants : lampe à incandescence, diode électroluminescente, lampe à fluorescence.

23. Dispositif selon la revendication 22, **caractérisé par le fait que** l'instrument optique comporte des éléments lumineux éclairant dans des gammes respectives de longueur d'onde différentes.

24. Dispositif selon l'une quelconque des revendications 21 à 23, **caractérisé par le fait que** l'instrument optique comporte plusieurs éléments lumineux et des moyens de commande (31) permettant d'alimenter sélectivement au moins une partie de ces éléments lumineux (17 ;17').

25. Dispositif selon l'une quelconque des revendications 21 à 24, **caractérisé par le fait que** l'instrument optique comporte des éléments lumineux (17 ; 17' ; 130) disposés circulairement.

26. Dispositif selon l'une quelconque des revendications 21 à 25, **caractérisé par le fait que** l'instrument optique comporte un logement (13) pour recevoir une ou plusieurs piles électriques (14).

27. Dispositif selon la revendication 26, **caractérisé par le fait que** le logement présente un axe sensiblement perpendiculaire à un axe d'observation (X) de la zone examinée.

28. Dispositif selon l'une quelconque des revendications précédentes, **caractérisé par le fait que** l'instrument optique comporte une vitre (35) permettant de comprimer la peau dans la zone examinée pour en chasser le sang.

29. Dispositif selon la revendication précédente, **caractérisé par le fait que** ladite vitre constitue un accessoire amovible.

30. Dispositif selon l'une quelconque des revendications précédentes, **caractérisé par le fait qu'**il comporte un atlas comprenant une pluralité d'images de référence (60 ; 70 ; 80).

31. Dispositif selon la revendication 30, **caractérisé par le fait que** les images sont disposées sur un support unique.

32. Dispositif selon l'une quelconque des revendications précédentes, **caractérisé par le fait que** les images de référence sont associées chacune à une indication alphanumérique (61 ; 71 ; 81 ; 91).

33. Dispositif selon l'une quelconque des revendications 1 à 29, **caractérisé par le fait qu'**il comporte un ordinateur (110) pour afficher des images de référence (90).

34. Dispositif selon l'une quelconque des revendications précédentes, **caractérisé par le fait que** l'instrument optique comporte un réticule (46).

35. Procédé pour évaluer le degré d'une caractéristique de la typologie corporelle, **caractérisé par le fait qu'**il comporte les étapes suivantes :
a) observer la peau ou les cheveux au moyen d'un instrument optique (10 ; 10' ; 40 ; 120) tel que défini dans l'une quelconque des revendications précédentes,
b) comparer l'image observée avec des images de référence (60 ; 70 ; 80 ; 90),
c) sélectionner une image de référence.

36. Procédé selon la revendication précédente, **caractérisé par le fait qu'**on observe le contraste entre la lumière qui comporte une composante de réflexion et une composante rétrodiffusée et la lumière qui comporte essentiellement une composante rétrodiffusée, en éclairant la zone examinée avec une lumière polarisée et en observant avec un analyseur.

37. Procédé selon la revendication 35 ou 36, **caractérisé par le fait que** les images de référence (90) sont affichées à l'écran d'un ordinateur (110).

38. Procédé selon la revendication 37, **caractérisé par le fait que** les images de référence (90) sont transmises à l'ordinateur depuis un serveur (100) par un réseau informatique, avant d'être affichées sur ledit écran.

39. Procédé selon l'une quelconque des revendications 35 à 38, **caractérisé par le fait qu'**il comporte en outre l'étape consistant à transmettre au serveur (100) une indication représentative de l'image de référence sélectionnée.

40. Procédé selon l'une quelconque des revendications 35 à 39, **caractérisé par le fait que** l'on effectue à l'aide du même instrument optique successivement ou simultanément au moins deux types d'observations, choisis parmi les suivants :
i) observation sous éclairage diffus,
ii) observation sous éclairage rasant,
iii) observation sous éclairage directif,
iv) observation par transillumination,
v) observation sous lumière polarisée avec un analyseur non croisé,
vi) observation sous lumière polarisée avec analyseur croisé,
vii) observation en comprimant la peau,
viii) observation sans comprimer la peau,
ix) observation en étirant la peau,
x) observation sans étirer la peau,
xi) observation en plissant la peau,
xii) observation sans plisser la peau.

41. Procédé selon l'une quelconque des revendications 34 à 39, **caractérisé par le fait que** l'on procède à une évaluation d'une caractéristique de la typologie corporelle, **par le fait que** l'on effectue un traitement, notamment cosmétique susceptible d'avoir une incidence sur ladite caractéristique, puis **par le fait que** l'on procède à une nouvelle évaluation pour détecter une éventuelle évolution de ladite caractéristique et déterminer l'efficacité du traitement.

42. Procédé selon la revendication 40, **caractérisé par le fait qu'**on observe par transillumination et **par le fait que** l'on mesure la distance à partir de laquelle la diffusion de la lumière n'est plus visible.
